# FASCICULE DE BREVET EUROPEEN

(11) **EP 0 621 024 B1**
(45) Date de publication et mention de la délivrance du brevet: **23.10.1996**
(21) Numéro de dépôt: 94400648.5
(22) Date de dépôt: 25.03.1994
(51) Int. Cl.: A61F 13/08

(54) **Article de contention à usage médical pour une jambe ou un bras**
Medizinische Kompressionseinrichtung für ein Bein oder einen Arm
Medical compression device for a leg or an arm

(30) Priorité: 02.04.1993 FR 9303904
(43) Date de publication de la demande: 26.10.1994
(73) Titulaire: COGNON-MORIN, F-86102 Chatellerault (FR)
(72) Inventeur: Arabeyre, Françoise, F-86100 Chatellerault (FR); Arabeyre, René, F-86100 Chatellerault (FR)
(74) Mandataire: Jaunez, Xavier

(56) Documents cités:
- DE-U- 8 217 651
- FR-A- 2 302 054
- US-A- 3 800 331

## Description

L'invention concerne les articles de contention à usage médical utilisés pour une jambe ou un bras, du type comportant une partie principale en tricot élastique servant à comprimer le membre sur lequel l'article est enfilé pour traiter une insuffisance veineuse ou lymphatique pour exercer une pression qui est dégressive du bas vers le haut de l'article.

Selon le type d'insuffisance concerné (ulcère, varices, oedème, phlébite, lymphoedème) et la zone affectée du corps du patient, on utilise en général pour une jambe un bas, un mi-bas ou une chaussette, et pour un bras un manchon dont la longueur varie selon le cas, ce manchon étant éventuellement équipé d'une épaulière.

L'état de la technique est illustré par les documents FR-A-2.671.485 ,FR-A-2.654.925 , FR-A-2.588.890, FR-A-2.587.617 et CH-A-675.201.

Ces différents articles de contention doivent, en plus de l'effet thérapeutique qu'ils produisent du fait de la compression exercée sur le membre concerné, être tolérés le mieux possible par le patient.

On a pu en effet constater que le patient déjà traumatisé par l'état dans lequel il se trouve, accepte de porter régulièrement l'appareil de contention qui lui est prescrit seulement si un certain confort lui est assuré, cette sensation de confort étant autant d'ordre physiologique (en cas de glissement et/ou de plissement de l'appareil par exemple) que psychologique (aspect traumatisant de moyens d'attache encombrants et/ou compliqués).

Le problème du glissement progressif de l'article est en particulier doublement important, car ce glissement est non seulement désagréable pour le patient (physiologiquement pour la sensation de gêne, et psychologiquement pour le caractère inesthétique qui en résulte), mais son effet thérapeutique est aussi amoindri.

Dans le cas particulier des bas de contention à usage médical (lorsque le patient ne peut pas ou ne veut pas porter un collant de contention), on utilise actuellement soit des moyens d'accrochage et de suspension inspirés de ceux de la lingerie traditionnelle (par exemple des jarretelles), soit des moyens de collage. Les jarretelles ou les bretelles constituent certes un moyen efficace mais, outre leur caractère obsolète, ces systèmes restent contraignants, gênants, et visibles en cas de vêtements ajustés ; la colle directe sur la peau, même lavable à l'eau, est quant à elle difficilement acceptée pour des raisons physiques et psychologiques bien compréhensibles.

Dans le cas de manchons conçus pour traiter un oedème du bras, en particulier dans le cadre d'intervention sur le sein et/ou de cobaltothérapie, on utilise traditionnellement un système d'attache comportant une bride passant sous un sein pour s'attacher sur le côté, ce qui a souvent un effet traumatisant pour la personne déjà choquée par les traitements qu'elle a subis.

On pourrait être tenté de s'inspirer des systèmes anti-dérapants que l'on trouve en bonneterie sur les bas de ville, basés sur le principe d'une bande supérieure à serrage énergique revêtue, du côté intérieur, d'une ou deux lignes circonférentielles en silicone.

Dans les bas de bonnetterie équipés d'une telle bande d'anti-glissement, la bande supérieure utilisée est élastique, et ses fibres constitutives sont choisies pour que ladite bande exerce sur la cuisse une pression largement supérieure (en général au moins 3 à 4 fois supérieure) à la très faible pression qu'exerce le bas proprement dit : le bas médical étant destiné à améliorer la circulation de retour, il ne saurait être question d'utiliser une bande ainsi conçue, qui constituerait une entrave en haut de cuisse, et précisément au niveau de la saphène. De plus, le système anti-dérapant par bande élastique utilisé pour les bas de bonneterie présente en général une caractéristique d'allongement à forte pente, par exemple correspondant à 5 à 7 % d'allongement par Newton exercé en traction, de sorte que cela induit de fortes variations de la pression exercée dans une taille de bas donnée selon les dimensions du tour de cuisse : ceci irait donc là encore à l'encontre de l'effet recherché avec un bas médical de contention, pour lequel il est absolument nécessaire d'éviter tout risque de strangulation du membre concerné.

De ce fait, l'enseignement des bas de bonneterie n'est a priori d'aucun secours pour résoudre le problème posé.

Ces tentatives peuvent être illustrées dans les trois documents qui suivent.

Le document DE-U-82 17 651.5, qui répresente le préambule de la revendication 1, décrit un bas médical dont la partie principale se prolonge supérieurement par une bande élastique de maintien. Cette bande de maintien est formée d'un ruban de galon élastique réalisé sur une machine à crochets, qui est revêtu, du côté intérieur, d'un motif réalisé en un matériau d'anti-glissement, conformément au préambule de la revendication 1. Le motif en matériau d'anti-glissement associé à la bande de maintien de ce bas médical est réalisé sous la forme de points ou de segments agencés de façon discontinue. Il est prévu que ce ruban présente d'une part la même caractéristique d'allongement que la partie principale du bas au voisinage dudit ruban, donc une caractéristique à forte pente (pour une représentation illustrant la force de traction en fonction du pourcentage d'allongement), et exerce d'autre part la même pression que cette partie principale.

Un tel ruban réalise donc un prolongement de l'action thérapeutique du bas, et induit un risque de strangulation, certes plus faible qu'avec un bas de bonneterie, mais suffisant pour qu'un bas de ce type soit considéré comme peu performant en bas médical de contention.

Le document FR-A-2.302.054 décrit un bas thérapeutique équipé d'une bande élastique de maintien fixée par surjet à la partie principale du bas. Cette bande de maintien est constituée par un tissu élastique revêtu intérieurement, pratiquement sur toute sa hauteur, d'un élastomère rainuré. Un tel revêtement, dont il est dit qu'il est non glissant, ne permet pas d'assurer un anti-glissement efficace, car l'adhérence à la peau est insuffisante avec le serrage autorisé (un serrage excessif induisant en effet un risque de strangulation). Ce document ne contient d'ailleurs aucun enseignement sur le choix d'une caractéristique d'allongement et/ou d'une pression exercée particulières pour la bande de maintien, mais seulement un enseignement visant à éviter l'effet de garot au niveau de la couture d'accrochage de ladite bande pour ne pas marquer la peau.

Le document US-A-3.800.331 décrit enfin un bas à pression uniforme (donc non dégressive) équipé d'une bande anti-glisse formée par une alternance de zones annulaires tricotées avec un fil élastique ressortant, et de zones de transition à fil vertical. Il est indiqué que le diamètre de la bande est choisi égal ou supérieur au diamètre de la partie principale du bas au voisinage de ladite bande. Avec un tel enseignement consistant à choisir la longueur de la bande (à plat) pour régler la pression exercée, on ne se préoccupe aucunement de la caractéristique d'allongement, de sorte que le serrage exercé par la bande peut être excessif. C'est sans doute pour cette raison qu'un tel bas n'est utilisable qu'en sur mesure, comme cela est précisé dans le document. Subsidiairement, la bande anti-glisse comporte, du côté intérieur, des fils élastiques nus proéminents, ce qui est une source d'inconfort (notamment du fait que les fils pincent les poils).

L'invention vise précisément à résoudre ce problème, en concevant un article de contention équipé de moyens efficaces d'anti-glissement.

L'invention a ainsi pour objet de réaliser un article de contention, notamment un bas médical, équipé de moyens d'anti-glissement à la fois efficaces, discrets, confortables et non-traumatisants.

Il s'agit plus particulièrement d'un article de contention à usage médical utilisé pour une jambe ou un bras, comportant une partie principale en tricot élastique servant à comprimer le membre sur lequel l'article est enfilé pour traiter une insuffisance veineuse ou lymphatique, laquelle partie principale se prolonge supérieurement par une bande, élastique de maintien formée d'une tresse élastique ou d'une dentelle élastique revêtue, du côté intérieur, d'un motif réalisé en un matériau d'anti-glissement, caractérisé en ce que les fibres constituant la tresse ou la dentelle de la bande de maintien sont choisies de telle façon que ladite bande présente à la fois une caractéristique d'allongement (force de traction en fonction du pourcentage d'allongement) à faible pente, et exerce sur le membre concerné une pression qui est inférieure à celle qu'exerce la partie principale au voisinage de la bande de maintien.

Il est intéressant de noter que les caractéristiques de la bande de maintien de l'article de contention à usage médical selon l'invention sont en totale opposition avec celles de la bande anti-dérapante des bas de bonneterie.

De préférence, le motif d'anti-glissement de la bande élastique de maintien est réalisé sous la forme d'au moins une bande, ligne ou succession de points jointifs, enduite en continu et s'étendant circonférentiellement sur la périphérie interne de ladite bande, avec une hauteur totale essentiellement comprise entre 1 et 6 cm.

A titre d'exemple avantageux, il est possible de prévoir que le motif d'anti-glissement est constitué par deux bandes superposées d'environ 1,5 cm de hauteur, ces deux bandes étant séparées par une portion non revêtue de la tresse ou dentelle élastique d'environ 1 cm de hauteur, ledit motif pouvant en outre être réalisé en silicone ou analogue déposé, en continu ou en discontinu, sur la périphérie interne de la bande élastique de maintien.

De préférence, encore, les fibres constituant la tresse ou la dentelle élastique sont choisies de telle façon que la caractéristique d'allongement de la bande élastique de maintien corresponde, à partir d'un allongement de 15 % sensiblement à 10 % d'allongement par Newton exercé en traction, et que la bande élastique de maintien exerce sur le membre concerné une pression essentiellement comprise entre 0,1 Po et 0,8 Po, où Po est la pression exercée sur ce membre par la partie principale au voisinage de cette bande de maintien.

Avantageusement alors, la tresse ou dentelle élastique de la bande élastique de maintien présente une longueur circonférentielle coupée en fonction de plusieurs tailles différentes, en particulier cinq tailles, et la pression exercée par cette bande de maintien est dans une fourchette qui est d'autant plus étroite que la taille est grande. En particulier, les valeurs de la pression exercée par la bande élastique de maintien sont de préférence respectivement comprises entre 0,2 Po et 0,6 Po, 0,4 Po et 0,75 Po, 0,45 Po et 0,8 Po, pour les trois tailles les plus courantes.

Il est par ailleurs intéressant que la tresse ou dentelle élastique soit réalisée à partir d'une fibre élastique et de fibres textiles du type continu ou texturé. En particulier, la fibre élastique est en élasthanne ou en élastodiène, et les fibres textiles sont en polyamide, en coton ou analogue.

De préférence enfin, la tresse élastique est tissée ou tricotée.

D'autres caractéristiques et avantages de l'invention ressortiront plus clairement à la lumière de la description qui va suivre et des dessins annexés, concernant un mode de réalisation particulier, en référence aux figures où :
- les figures 1 et 2 illustrent deux articles de contention conformes à l'invention, respectivement sous forme d'un bas de jambe et d'un manchon de bras, avec leur bande élastique de maintien ;
- les figures 3a, 3b, et 3c illustrent, à plus grande échelle, trois exemples d'agencement du motif d'anti-glissement prévu sur la périphérie interne de la bande élastique de maintien ;
- la figure 4 est un diagramme d'allongement, illustrant en trait plein la caractéristique à faible pente de la bande élastique de maintien conforme à l'invention, et, en pointillés et en traits mixtes, la caractéristique à forte pente de deux bandes typiques utilisées comme système antidérapant pour des bas de bonneterie.

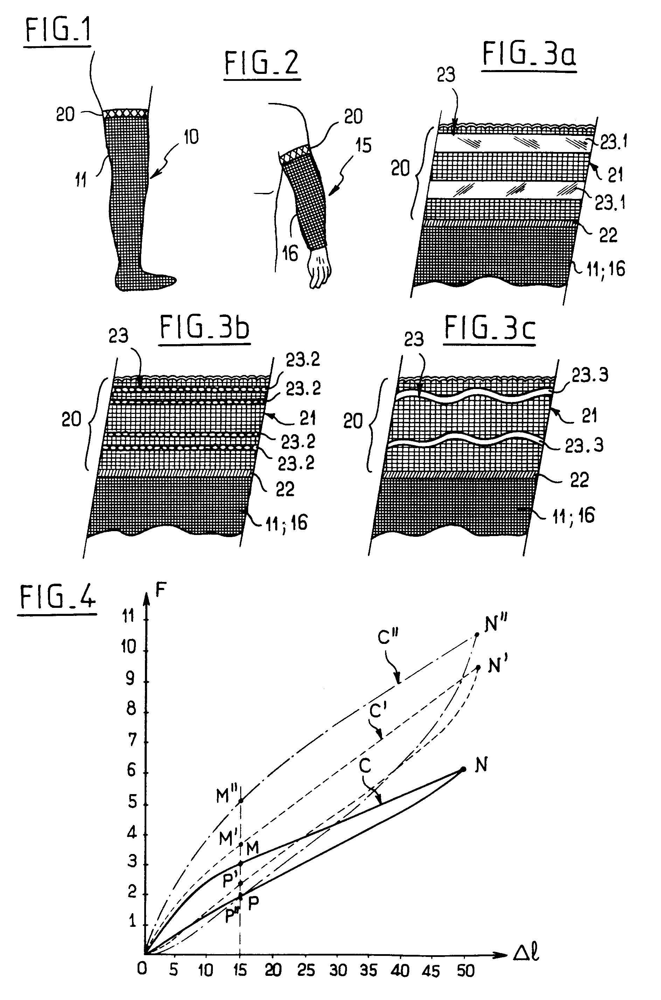

Les figures 1 et 2 illustrent deux articles de contention conformes à l'invention, et l'on distingue ainsi, sur la figure 1, un bas de jambe 10 et, sur la figure 2, un manchon de bras 15.

Conformément à la pratique habituelle en la matière, chaque article de contention à usage médical 10 ou 15 comporte une partie principale respectivement 11 ou 16 en tricot élastique, servant à comprimer le membre sur lequel l'article est enfilé pour traiter une insuffisance veineuse ou lymphatique, la pression exercée étant dégressive du bas vers le haut de l'article.

Conformément à une caractéristique essentielle de l'invention, la partie principale 11 ou 16 de l'article de contention à usage médical 1O ou 15 se prolonge supérieurement par une bande élastique de maintien 2O, dont la structure particulière va être décrite ci-après en détail, cette bande élastique de maintien étant capable d'éviter le glissement de la partie supérieure de l'article de contention 10 ou 20, et par suite le plissement de cet article, tout en écartant tout risque de strangulation du membre concerné qui est comprimé. La bande élastique de maintien 2O est ainsi formée d'une tresse élastique ou d'une dentelle élastique qui est revêtue, du côté intérieur, d'un motif réalisé en un matériau d'anti-glissement, et les fibres constituant la tresse ou la dentelle de cette bande de maintien sont choisies de façon que ladite bande de maintien présente des caractéristiques prédéterminées de souplesse et de pression exercée.

Sur les figures 3a, 3b, 3c, on a illustré trois exemples d'agencement possibles du motif d'anti-glissement 23 agencé circonférentiellement sur la périphérie interne de la bande élastique de maintien 2O.

Sur la figure 3a, on distingue ainsi une tresse élastique 21, qui est raccordée par une couture circonférentielle 22 à la partie principale 11 ou 16 de l'article de contention concerné. On a illustré ici une tresse élastique, mais il pourra naturellement s'agir en variante d'une dentelle élastique. Il va de soi que la bande élastique de maintien 2O, dont on ne distingue ici qu'une partie de sa longueur, concerne tout le tour du membre concerné. La tresse élastique 21 est revêtue, du côté intérieur que l'on a représenté sur la figure 3a, d'un motif 23 réalisé en un matériau d'anti-glissement, ce motif étant de préférence réalisé en silicone ou analogue, ici déposé en continu sur la périphérie interne de la bande élastique de maintien 2O. Sur la figure 3a, on a illustré un motif d'anti-glissement 23 constitué par deux bandes superposées 23.1, qui sont ici rectilignes.

Sur les figures 3b et 3c, on a illustré deux variantes de cette bande élastique de maintien 2O, dans lesquelles le motif d'anti-glissement 23 est respectivement réalisé sous la forme de successions de points jointifs 23.2, ou de lignes ondulées superposées 23.3, enduites en continu. On a illustré ici, dans le cas d'une succession de points, un ensemble de quatre lignes de points 23.2, et, pour les lignes superposées de la figure 3c, on a illustré deux lignes sinusoïdales superposées 23.3.

Les expériences menées par la demanderesse ont permis de constater qu'il était avantageux de prévoir que la hauteur totale du motif d'anti-glissement 23 de la bande élastique de maintien 20 est de préférence essentiellement comprise entre 1 et 6 cm. En effet, si la hauteur de bande est trop faible, on risque d'avoir un effet de strangulation tout à fait indésirable dans le cadre d'un article de contention à usage médical, et si cette hauteur est trop grande, la bande élastique de maintien atteint un degré d'inconfort tel que le patient ne voudra pas porter l'article concerné.

Sur la figure 3a, on a par exemple illustré le cas de deux bandes superposées 23.1 constituant le motif d'anti-glissement 23, chaque bande présentant une hauteur d'environ 1,5 cm, et ces deux bandes étant séparées par une portion non revêtue de la tresse élastique 21 sur une hauteur d'environ 1 cm, de sorte que le motif d'anti-glissement présente une hauteur totale de l'ordre de 3 cm. Sur la figure 3b, on a illustré un ensemble de quatre lignes superposées 23.2, constituées par des successions de points jointifs, la hauteur de chaque ligne individuelle étant d'environ 0,5 cm, ce qui donne une hauteur de motif d'anti-glissement d'environ 2 cm. Sur la figure 3c, on a illustré deux lignes sinusoïdales 23.3 superposées, dont la hauteur individuelle est de l'ordre de 0,5 cm, de sorte que le motif d'anti-glissement présente une hauteur totale de l'ordre de 1 cm.

Il va de soi que l'on pourra envisager encore d'autres types de motifs d'anti-glissement en modifiant les exemples qui viennent d'être décrits, notamment des motifs discontinus sous forme de bandes ou lignes agencées en tronçons disjoints s'étendant circonférentiellement (ce qui permet de moins brider le membre concerné que les bandes ou lignes continues).

Le motif d'anti-glissement prévu sur le coté intérieur de la tresse ou de la dentelle élastique ne génère à lui seul pratiquement aucune force de retour lorsqu'une traction est exercée sur la bande. De ce fait, lorsqu'il est question des caractéristiques d'allongement de la bande élastique de maintien de l'article de contention selon l'invention, les caractéristiques recherchées pour ladite bande doivent être données par les fibres constituant la tresse ou la dentelle élastique concernées.

Conformément à une caractéristique de l'invention, les fibres constituant la tresse ou la dentelle de la bande élastique de maintien sont choisies de telle façon que ladite bande présente à la fois une caractéristique d'allongement à faible pente, et exerce sur le membre concerné une pression qui est inférieure à celle qu'exerce la partie principale au voisinage de cette bande de maintien.

La bande élastique de maintien se différencie ainsi radicalement des bandes déjà utilisées comme moyen antidérapant pour des bas de bonneterie.

Afin de mieux faire comprendre combien ces différences sont sensibles, tant pour la souplesse de la bande que pour la pression exercée sur le membre concerné, on va donner ci-après, à titre d'exemple seulement, quelques indications comparatives entre une bande élastique de maintien d'un article de contention à usage médical conforme à l'invention, et des bandes élastiques de même dimensions du type de celle que l'on utilise comme moyen anti-dérapant pour les bas de bonneterie.

On va tout d'abord s'intéresser en se référant à la figure 4, à la caractéristique d'allongement des bandes concernées.

La figure 4 est un diagramme d'allongement illustrant les variations de la force de traction exercée sur une bande élastique en fonction de l'allongement de cette bande. En abscisses, on a porté le pourcentage d'allongement Δl par rapport à la longueur initiale (au repos) de la bande concernée, et en ordonnées la force de traction exercée sur cette bande avec une graduation en Newton. Avec une bande élastique de maintien conforme à l'invention, on obtient une courbe C dont la zone utile MN est linéaire et présente une pente faible lors de la montée en traction. Au relâchement de la force appliquée, le tronçon de courbe passe par un point P pour rejoindre l'origine du diagramme, en raison de l'hystérésis de la bande élastique. On peut ainsi observer que la caractéristique d'allongement de la bande élastique de maintien conforme à l'invention correspond, à partir d'un allongement de 15 % (point M), sensiblement à 10 % d'allongement par Newton exercé en traction.

Sur cette même figure 4, on a également tracé deux courbes typiques C' et C'' concernant des bandes élastiques d'un type courant équipant des bas de bonneterie. Pour chacune de ces courbes, on retrouve une zone utile essentiellement linéaire M'N' ou M''N'', mais les pentes concernées sont beaucoup plus fortes que celle du tronçon MN correspondant à une bande élastique selon l'invention.

Grâce à cette faible pente pour la caractéristique d'allongement, on obtient de faibles variations de pression dans une gamme de taille donnée, ce qui procure, pour le patient, une sensation de souplesse qui permet une grande tolérance entre les variations dimensionnelles du membre concerné en fonction de la taille de l'article de contention retenu (par exemple, en cas de cheville de petite taille et de haut de cuisse de grande taille), et aussi en fonction des variations de la circonférence de ce membre (on sait par exemple que la circonférence de la cuisse varie sensiblement lors de la marche et au cours de la journée). Il est ainsi possible de réaliser un article de contention à usage médical qui est dégressif pour la pression qu'il applique sur le membre concerné, en suivant une loi prédéterminée, la bande élastique de maintien qui prolonge supérieurement cet article n'affectant aucunement la souplesse pour une taille donnée, tout en prolongeant la dégressivité de la pression exercée. En effet, on détermine dans la pratique la pression maximum que l'on veut exercer en partie haute du membre concerné en fonction de la pression exercée en partie basse, de façon à obtenir l'effet thérapeutique désiré au regard d'une insuffisance veineuse ou lymphatique.

Il est à noter que la faible pente précitée est en particulier inférieure à la pente de la caractéristique d'allongement de la partie principale du bas au voisinage de la bande de maintien, cette dernière pente étant dans la pratique conforme à la pente des tronçons M'N' ou M''N'' tracés sur la figure 4.

La bande élastique de maintien de l'article de contention selon l'invention doit également satisfaire à des critères de pression exercée, dans la mesure où, ainsi que cela a été dit plus haut, la bande de maintien doit exercer sur le membre concerné une pression qui est inférieure à celle qu'exerce la partie principale de l'article de contention au voisinage de cette bande de maintien.

Les essais menés par la demanderesse tendent à montrer que l'on obtient de bons résultats en choisissant une pression P essentiellement comprise entre 0,1 Po et O,8 Po, où Po est la pression exercée sur ce membre par la partie principale 11 ou 16 au voisinage de cette bande de maintien. En particulier, la tresse ou la dentelle élastique 21 de la bande élastique de maintien 2O présentera une longueur circonférentielle coupée en fonction de plusieurs tailles différentes, par exemple de cinq tailles, et la pression P exercée par cette bande de maintien sera alors dans une fourchette qui est d'autant plus étroite que la taille est grande.

A titre indicatif, pour les trois tailles les plus courantes généralement utilisées, c'est-à-dire les tailles qui correspondent à un tour de cuisse respectivement de 46 à 48 cm, de 49 à 53 cm et de 54 à 58 cm, on choisira les fibres constituant la tresse ou la dentelle élastique de la bande de maintien de telle façon que les valeurs de la pression P exercée par cette bande élastique soient de préférence respectivement comprises entre 0,2 Po et 0,6 Po, 0,4 Po et 0,75 Po, 0,45 Po et 0,8 Po pour les trois tailles précitées.

La tresse ou dentelle élastique 21 sera de préférence réalisée à partir d'une fibre élastique et de fibres textiles du type continu ou texturé. La fibre élastique pourra être en élasthanne, qui est une fibre élastomère constituée pour au moins 85 % de sa masse de polyuréthanne segmentaire, ou d'élastodiène, qui est une fibre élastomère constituée de polyisoprène naturel ou synthétique, soit d'un ou de plusieurs diènes polymérisés avec ou sans un ou plusieurs monomères vinyliques. A titre indicatif, on pourra choisir pour cette fibre élastique une masse linéique de 10 à 160 décitex. Les fibres textiles sont quant à elles choisies de préférence en polyamide, en coton, ou analogue, lesdites fibres pouvant être du type continu ou texturé. Dans la pratique, la tresse élastique 21 sera tissée ou tricotée.

On est ainsi parvenu à réaliser un article de contention à usage médical, notamment un bas médical ou un manchon médical, équipé de moyens d'anti-glissement à la fois efficaces, discrets, confortables et non traumatisants.

Dans le cas des jambes, l'invention permet le port de bas de contention qui sont plus hygiéniques que les collants, et qui sont en outre particulièrement indiqués dans le cas particulier des femmes enceintes. L'article de contention, grâce à son confort qui le fait presque oublier par le patient qui le porte, pourra alors être prescrit même pendant des périodes estivales. Dans le cas de l'oedème du bras, l'invention permet d'éviter le traumatisant système d'attache, ce qui constitue un avantage très important pour les personnes qui sont déjà choquées par une intervention du sein ou une cobaltothérapie.

L'invention n'est pas limitée aux modes de réalisation qui viennent d'être décrits, mais englobe au contraire toute variante reprenant, avec des moyens équivalents, les caractéristiques essentielles énoncées plus haut.

## Revendications

1. Article de contention à usage médical utilisé pour une jambe ou un bras, comportant une partie principale (11 ; 16) en tricot élastique servant à comprimer le membre sur lequel l'article est enfilé pour traiter une insuffisance veineuse ou lymphatique, laquelle partie principale se prolonge supérieurement par une bande élastique de maintien (20) formée d'une tresse élastique ou d'une dentelle élastique (21) revêtue, du côté intérieur, d'un motif (23) réalisé en un matériau d'anti-glissement, caractérisé en ce que les fibres constituant la tresse ou la dentelle (21) de la bande de maintien (20) sont choisies de telle façon que ladite bande présente à la fois une caractéristique d'allongement (force de traction en fonction du pourcentage d'allongement) à faible pente, et exerce sur le membre concerné une pression qui est inférieure à celle qu'exerce la partie principale (11 ; 16) au voisinage de la bande de maintien (20).

2. Article de contention selon la revendication 1, caractérisé en ce que le motif d'anti-glissement (23) de la bande élastique de maintien (20) est réalisé sous la forme d'au moins une bande (23.1), ligne (23.3) ou succession de points jointifs (23.2), enduite en continu et s'étendant circonférentiellement sur la périphérie interne de ladite bande, avec une hauteur totale essentiellement comprise entre 1 et 6 cm.

3. Article de contention selon la revendication 2, caractérisé en ce que le motif d'anti-glissement (23) est constitué par deux bandes superposées (23.1) d'environ 1,5 cm de hauteur, ces deux bandes étant séparées par une portion non revêtue de la tresse ou dentelle élastique (21) d'environ 1 cm de hauteur.

4. Article de contention selon la revendication 2 ou la revendication 3, caractérisé en ce que le motif d'anti-glissement (23) est réalisé en silicone ou analogue déposé, en continu ou en distontinu, sur la périphérie interne de la bande élastique de maintien (20).

5. Article de contention selon l'une des revendications 1 à 4, caractérisé en ce que les fibres constituant la tresse ou la dentelle élastique (21) sont choisies de telle façon que la caractéristique d'allongement de la bande élastique de maintien (20) corresponde, à partir d'un allongement de 15 %, sensiblement à 10 % d'allongement par Newton exercé en traction, et que la bande élastique de maintien (20) exerce sur le membre concerné une pression (P) essentiellement comprise entre 0,1 Po et 0,8 Po, où Po est la pression exercée sur ce membre par la partie principale (11; 16) au voisinage de cette bande de maintien.

6. Article de contention selon la revendication 5, caractérisé en ce que la tresse ou dentelle élastique (21) de la bande élastique de maintien (2O) présente une longueur circonférentielle coupée en fonction de plusieurs tailles différentes, en particulier cinq tailles, et la pression (P) exercée par cette bande de maintien est dans une fourchette qui est d'autant plus étroite que la taille est grande.

7. Article de contention selon la revendication 6, caractérisé en ce que les valeurs de la pression (P) exercée par la bande élastique de maintien (20) sont de préférence respectivement comprises entre 0,2 Po et 0,6 Po, 0,4 Po et 0,75 Po, 0,45 Po et 0,8 Po, pour les trois tailles les plus courantes.

8. Article de contention selon l'une des revendications 1 à 7, caractérisé en ce que la tresse ou dentelle élastique (21) est réalisée à partir d'une fibre élastique et de fibres textiles du type continu ou texturé.

9. Article de contention selon la revendication 8, caractérisé en ce que la fibre élastique est en élasthanne ou en élastodiène, et les fibres textiles sont en polyamide, en coton, ou analogue.

10. Article de contention selon la revendication 8 ou la revendication 9, caractérisé en ce que la tresse élastique (21) est tissée ou tricotée.

## Claims

1. A remedial support appliance for medical use on a leg or an arm, comprising a main portion (11 ; 16) of elasticated knit serving to compress the limb on which the appliance is worn for the purpose of treating venous or lymphatic insufficiency, which main portion is extended upwards by an elasticated garter band (20), which is formed by an elasticated braid or an elasticated lace (21) covered on the inside with a pattern (23) made of an antislip material, characterized in that the fibers constituting the braid or the lace (21) of the garter band (20) are selected in such a manner that said band presents an elongation characteristic [traction force as a function of percentage elongation] of a shallow slope and simultaneously exerts pressure on the limb in question that is less than the pressure exerted by the main portion (11; 16) in the vicinity of the garter band (20).

2. A remedial support appliance according to claim 1, characterized in that the antislip pattern (23) of the elasticated garter band (20) is implemented in the form of at least one band (23.1), line (23.3), or succession of touching stitches (23.2), continuously disposed on and extending circumferentially around the inside periphery of said garter band, with the total height of the antislip pattern lying essentially in the range 1 cm to 6 cm.

3. A remedial support appliance according to claim 2, characterized in that the antislip pattern (23) is constituted by two superposed bands (23.1) each about 1.5 cm high, the two bands being separated by an uncovered portion of the elasticated lace or braid (21) that is about 1 cm high.

4. A remedial support appliance according to claim 2 or claim 3, characterized in that the antislip pattern (23) is made of silicone or the like deposited continuously or discontinuously on the inside periphery of the elasticated garter band (20).

5. A remedial support appliance according to any one of claims 1 to 4, characterized in that the fibers constituting the elasticated lace or braid (21) are selected in such a manner that the elongation characteristic of the elasticated garter band (20) corresponds, above an elongation of 15%, substantially to 10% elongation per Newton exerted in traction, and that the elasticated garter band (20) exerts pressure (P) on the limb in question that lies essentially in the range 0.1 P0 to 0.8 P0 where P0 is the pressure exerted on the limb by the main portion (11; 16) of the appliance in the vicinity of said garter band.

6. A remedial support appliance according to claim 5, characterized in that the elasticated lace or braid (21) of the elasticated garter band (20) is cut to a circumferential length that is determined as a function of various different sizes, in particular five sizes, and the pressure (P) exerted by said garter band lies in a range that narrows with increasing size.

7. A remedial support appliance according to claim 6, characterized in that the values of pressure (P) exerted by the elasticated garter band (20) lie preferably in the following ranges 0.2 P0 to 0.6 P0, 0.4 P0 to 0.75 P0, and 0.45 P0 to 0.8 P0 respectively for the three commonest sizes.

8. A remedial support appliance according to any one of claims 1 to 7, characterized in that the elasticated lace or braid (21) is made from an elasticated fiber and from textile fibers of textured or continuous type.

9. A remedial support appliance according to claim 8, characterized in that the elasticated fiber is an elasthanne or an elastodiene, and the textile fibers are made of polyamide, cotton or the like.

10. A remedial support appliance according to claim 8 or claim 9, characterized in that the elasticated braid (21) is woven or knitted.

## Patentansprüche

1. Medizinische Kompressionseinrichtung für einen Bein oder einen Arm, umfassend einen Hauptabschnitt (11; 16) aus elastischem Trikot, der zur Kompression des Gliedes dient, auf das die Kompressionseinrichtung zur Behandlung einer Veneninsuffizienz oder lymphatischen Insuffizienz aufgeschoben wird, wobei der Hauptabschnitt nach oben durch ein elastisches Halteband (20) verlängert ist, das von einem elastischen Geflecht oder einem elastischen Spitzengewebe (21) gebildet ist, das auf seiner Innenseite mit einem aus einem Antigleitmaterial hergestellten Muster (23) bedeckt ist, dadurch **gekennzeichnet**, daß die das Geflecht oder das Spitzengewebe (21) des Haltebandes (20) bildenden Fasern derart gewählt sind, daß das genannte Band gleichzeitig eine Dehnungskennlinie (Zugkraft als Funktion der prozentualen Längung) mit geringer Steigung hat und auf das betreffende Glied einen Druck ausübt, der geringer als der von dem Hauptabschnitt (11; 16) nahe dem Halteband (20) ausgeübte Druck ist.

2. Kompressionseinrichtung nach Anspruch 1, dadurch **gekennzeichnet**, daß das Antigleitmuster (23) des elastischen Haltebandes in Form mindestens eines Streifens (23.1), einer Linie (23.3) oder einer Folge von nebeneinanderliegenden Punkten (23.2) gebildet ist, die kontinuierlich aufgetragen sind und sich in Umfangsrichtung über die innere Umfangsfläche des genannten Bandes erstrecken mit einer Gesamthöhe, die im wesentlichen zwischen 1 und 6 cm beträgt.

3. Kompressionseinrichtung nach Anspruch 2, dadurch **gekennzeichnet**, daß das Antigleitmuster (23) von zwei übereinanderliegenden Streifen (23.1) mit einer Höhe von ca. 1,5 cm gebildet ist, wobei die beiden Streifen durch einen unbedeckten Teil des elastischen Geflechts oder Spitzengewebes (21) mit einer Höhe von ca. 1 cm getrennt sind.

4. Kompressionseinrichtung nach Anspruch 2 oder 3, dadurch **gekennzeichnet**, daß das Antigleitmuster (23) aus Silikon oder einem ähnlichen Material hergestellt ist, das kontinuierlich oder diskontinuierlich auf der Innenumfangsfläche des elastischen Haltebandes (20) aufgetragen ist.

5. Kompressionseinrichtung nach einem der Ansprüche 1 bis 4, dadurch **gekennzeichnet**, daß die das elastische Geflecht oder Spitzengewebe (21) bildenden Fasern so ausgewählt sind, daß die Dehnungskennlinie des elastischen Haltebandes (20) von einer Dehnung von 15% an im wesentlichen 10% Längung pro Newtonzugkraft entspricht und daß das elastische Halteband (20) auf das betreffende Glied einen Druck (P) ausübt, der im wesentlichen zwischen 0,1 P0 und 0,8 P0 beträgt, wobei P0 der Druck ist, der auf das Glied von dem Hauptabschnitt (11; 16) nahe diesem Halteband ausgeübt wird.

6. Kompressionseinrichtung nach Anspruch 5, dadurch **gekennzeichnet**, daß das elastische Geflecht oder Spitzengewebe (21) des elastischen Haltebandes (20) eine Umfangslänge hat, die in Abhängigkeit mehrerer verschiedener Abmessungen, insbesondere fünf Abmessungen geschnitten ist und daß der von diesem Halteband ausgeübte Druck (P) in einem Bereich liegt, der um so schmaler ist, je größer die Abmessung ist.

7. Kompressionseinrichtung nach Anspruch 6, dadurch **gekennzeichnet**, daß die von dem elastischen Halteband (20) ausgeübten Druckwerte vorzugsweise zwischen 0,2 P0 und 0,6 P0, 0,4 P0 und 0, 75 P0, 0,45 P0 und 0,8 P0 für die drei häufigsten Abmessungen liegen.

8. Kompressionseinrichtung nach einem der Ansprüche 1 bis 7, dadurch **gekennzeichnet**, daß das elastische Geflecht oder Spitzengewebe (21) aus einer elastischen Faser und textilen Endlosfasern oder texturierten Fasern hergestellt ist.

9. Kompressionseinrichtung nach Anspruch 8, dadurch **gekennzeichnet**, daß die elastische Faser aus Elasthan oder Elastodien besteht und daß die textilen Fasern aus Polyamid, Baumwolle oder dergleichen bestehen.

10. Kompressionseinrichtung nach Anspruch 8 oder 9, dadurch **gekennzeichnet**, daß das elastische Geflecht (21) gewebt oder gestrickt ist.
